## Europäisches Patentamt

## European Patent Office

## Office européen des brevets

(11) Veröffentlichungsnummer: **0 160 711**
**B1**

(12) **EUROPÄISCHE PATENTSCHRIFT**

(45) Veröffentlichungstag der Patentschrift:
**07.12.88**

(51) Int. Cl.⁴: **A 61 M 5/24**

(21) Anmeldenummer: **84104961.2**

(22) Anmeldetag: **03.05.84**

(54) Injektionsspritze.

(43) Veröffentlichungstag der Anmeldung:
**13.11.85 Patentblatt 85/46**

(45) Bekanntmachung des Hinweises auf die Patenterteilung:
**07.12.88 Patentblatt 88/49**

(84) Benannte Vertragsstaaten:
**AT BE CH DE FR GB IT LI LU NL SE**

(56) Entgegenhaltungen:
**DE-A- 2 618 430**
**US-A- 3 391 695**
**US-A- 3 583 399**
**US-A- 3 811 441**

(73) Patentinhaber: **Bünder Glas GmbH,**
**Erich-Martens-Strasse 26-32, D-4980 Bünde 1 (Westf.)**
**(DE)**

(72) Erfinder: **Theiling, Manfred, Fichtestrasse 15,**
**D-4980 Bünde 11 (DE)**

(74) Vertreter: **Hanewinkel, Lorenz, Dipl.-Phys., Patentanwalt**
**Ferrariweg 17a, D-4790 Paderborn (DE)**

**Beschreibung**

Die Erfindung bezieht sich auf eine Injektionsspritze gemäß dem ersten Teil von Anspruch 1.

Bei bekannten, auf dem Markt befindlichen Injektionsspritzen ist der Luer-Lock-Anschluß mit seinem hülsenförmigen Innengewinde teilweise mit einem nachträglich montierten Konus ausgerüstet. Diese Luer-Lock-Ausführung gewährleistet keine sichere Verbindung zwischen Kanüle und Luer-Lock-Konus durch das kanülenseitige Kupplungsstück, welches mit dem Gewindeteil zusammenwirkt, da beim Eindrehen des Kupplungsstückes in den Gewindegang der Konus einseitig versetzt werden kann und dadurch keine feste und dichte Verbindung zwischen dem Konus und dem Kupplungsstück der Kanüle mehr möglich ist, so daß zwischen diesen beiden Teilen der Ampulleninhalt austreten kann.

Weiterhin ist der Konus des am Spritzenkörper angeordneten Luer-Lock-Anschlusses mit einer Überleitungskanüle ausgestattet, durch die eine Dichtungsmembrane der Zylinderampulle beim Einschieben derselben in Richtung Konus durchstoßen wird, damit ein Übergang vom Ampullen-Innenraum zur Kanüle geschaffen wird. Durch dieses Durchstoßen der Dichtungsmembrane werden oft winzige Teilchen der Membrane ausgestanzt, so daß keine fragmentationslose Verbindung zwischen Kanüle und Zylinderampulle erfolgen kann.

Aufgabe der Erfindung ist es, eine nach der eingangs genannten Art aufgebaute Injektionsspritze mit Luer-Lock-Anschluß dahingehend zu verbessern, daß sie eine perforations- und somit fragmentationslose Verbindung mit dichter und hoher Verbindungsfestigkeit zwischen Kanüle und Luer-Lock-Konus ermöglicht und als vorgefüllte Einmalspritze einfach und sicher zu handhaben ist.

Diese Aufgabe wird erfindungsgemäß durch eine Injektionsspritze gemäß Anspruch 1 gelöst.

Der an der Zylinderampulle festgelegte Konus des Luer-Lock-Anchlusses ist vorzugsweise abgestuft ausgebildet und hat einen in eine konische, die Widerlagerfläche bildende Bohrung des Bodens des Gewindeteiles einfassenden Einsteckkonus als Anlagefläche und einen sich daran anschließenden und aus dem Gewindeteil axial herausragenden Aufsteckkonus, auf dem der Verschlußstopfen abnehmbar lagert und auf den das Kupplungsstück der Kanüle mit einem Dichtkonus aufsetzbar ist.

Der im Oberbegriff des Patentanspruches 1 angeführte Spritzenkörper mit Luer-Lock-Anschluß und eingesetzter Ampulle ist z.B. aus der DE-A-26 18 430 bekannt geworden.

Auf dem dem Luer-Lock-Anschluß gegenüberliegenden Längenende des Spritzenkörpers ist eine Fixierhülse mit angeformter Fingerauflage durch Schrauben, Bajonettverschluß, Rastverbindung oder dergleichen festlegbar, die mit einer Druckfläche gegen die Stirnfläche der in den Spritzenkörper eingesetzten, gefüllten Zylinderampulle einwirkt und diese in Axialrichtung festhält.

Dadurch wird erreicht, daß sowohl die Längentoleranz der Zylinderampulle überbrückt und darüber hinaus in jedem Falle das mit der Zylinderampulle verbundene Konusteil fest in den vordersten Teilbereich des Spritzenkörpers (Außenmantels) eingepreßt und lagefixiert wird.

Weitere Merkmale der Erfindung sind aus den übrigen Unteransprüchen zu entnehmen.

Der Gegenstand der Erfindung erstreckt sich nicht nur auf die Merkmale der einzelnen Ansprüche, sondern auch auf deren Kombination.

Bei der erfindungsgemäßen Injektionsspritze ist der Luer-Lock-Anschluß zweiteilig ausgebildet, indem der Konus fest an der Zylinderampulle und das Gewindeteil an dem Spritzenkörper vorgesehen worden ist. Die befüllte Zylinderampulle wird durch eine Anlagefläche im Bereich des Gewindeteiles und durch eine auf den Spritzenkörper festlegbare Fixierhülse mit Fingerauflage in Axialrichtung lagegesichert in dem Spritzenkörper gehalten. Das Kupplungsstück der Kanüle ist bei Verwendung der Spritze auf den ampullenseitigen Konus mit einm Dichtkonus aufsteckbar und wird dann in das Gewinde des Spritzenkörpers eingeschraubt, wobei das Kupplungsstück den ampullenseitigen Konus fest in den Dichtkonus des Kupplungsstückes hineinzieht und eine sehr dichte und feste Verbindung schafft.

Der Konus der Zylinderampulle ist mit einem axialen Verbindungskanal ausgestattet, der durch einen abnehmbaren Verschlußstopfen verschlossen ist.

Beim Befestigen der Kanüle wird dieser Verschlußstopfen abgenommen, und dann ist sofort eine Verbindung zwischen Zylinderampullen-Innenraum und Kanüle hergestellt, so daß kein Dichtteil der Zylinderampulle durchstoßen werden muß und dadurch eine perforations- und fragmentationslose Verbindung zwischen Kanüle und Zylinderampulle erfolgt. Die durch den Verschlußstopfen verschlossene, gefüllte Zylinderampulle ist einerseits in dem Spritzenkörper lagesicher gehalten und durch den zweigeteilten Luer-Lock-Anschluß einfach und sicher sowie sehr dicht mit der Kanüle verbindbar.

Die erfindungsgemäße Injektionsspritze stellt eine zweiteilige, leicht und sicher zu handhabende, vorgefüllte Einmalspritze dar, die insbesondere in der Humanmedizin eingesetzt werden kann.

Anhand der Zeichnung wird nachfolgend ein Ausführungsbeispiel gemäß der Erfindung näher erläutert. Es zeigt:

Fig. 1 eine Seitenansicht im teilweisen Schnitt und in Explosionsdarstellung einer Injektionsspritze, bestehend aus Kanüle mit Kupplungsstück, Spritzenkörper mit Luer-Lock-Gewinde, Zylinderampulle mit Luer-Konus, Fixierhülse mit Fingerauflage und auf den Konus der Zylinderampulle abnehmbar aufsteckbaren Verschlußstopfen,

Fig. 2 einen Längsschnitt durch die zusammengesetzte Injektionsspritze,

Fig. 3 einen Längsschnitt durch den Gewindebereich des Luer-Lock-Anschlusses am Spritzenkörper,

Fig. 4 eine Seitenansicht des Konusses des Luer-Konus-Anschlusses an der Zylinderampulle,

Fig. 5 einen Längsschnitt durch die mittels Bajonettverschluß am Spritzenkörper gehaltene Fixierhülse mit Fingerauflage und mit Druckfläche bzw. Druckfeder,

Fig. 6 bis 8 Draufsichten auf die Fixierhülse mit unterschiedlichen Fingerauflagen.

Die erfindungsgemäße Injektionsspritze ist als Einmalspritze vorgesehen und setzt sich aus einer Kanüle 1 mit Kupplungsstück 2, einem Spritzenkörper 3, einer befüllten Zylinderampulle 4 und einer Fixierhülse 5 mit Fingerauflage 6 sowie einem Verschlußstopfen 7 für die gefüllte Zylinderampulle 4 zusammen.

Der Spritzenkörper 3 und die Zylinderampulle 4 bilden erfindungsgemäß zusammen einen zweiteiligen Luer-Lock-Anschluß für die Kanüle 1, die mittels ihres Kupplungsstückes 2 sowohl mit der Zylinderampulle 4 als auch mit dem Spritzenkörper 3 lösbar, aber dicht und sicher verbindbar ist.

Der Spritzenkörper 3 ist von einem zylindrischen, im Querschnitt kreisförmigen Rohr aus Kunststoff, vorzugsweise durchsichtigem Kunststoff, gebildet und hat an einem Längenende einen Teil eines Luer-Lock-Anschlusses, nämlich das Gewindeteil, welches sich aus einer Hülse 8 mit Innengewinde 9 (nach Norm) zusammensetzt. Dieses Gewindeteil 8, 9 ist Teil des rohrförmigen Spritzenkörpers 3, jedoch gegenüber der übrigen Spritzenkörperwandung nach innen hin in der Wanddicke vergrößert (verdickt).

Das Gewindeteil 8, 9 ist weiterhin topfartig ausgebildet, so daß es in der in Spritzenkörper-Axialrichtung verlaufend einen Teilbereich der Spritzenkörperlänge einnimmt und mit seinem Topfboden 8a eine Widerlagerfläche für die in den Spritzenkörper 3 eingesetzte Zylinderampulle 4 mit dem Anlageteil 14 bildet. Die Zylinderampulle 4 wird mit ihrer gesamten Länge vom Spritzenkörper 3 aufgenommen und ist an ihrem einen Längenende mit dem zweiten Teil des Luer-Lock-Anschlusses, nämlich dem Konus 10, ausgestattet, so daß ein Teil 8, 9 des Luer-Lock-Anschlusses am Spritzenkörper 3 und der zweite Teil 10 an der Zylinderampulle 4 vorgesehen ist. Dieser Konus 10 ist in das eine Längenende (den Ampullenhals 4a) eingesteckt und liegt auf der Stirnseite dieses Ampullenhalses 4a unter Zwischenschaltung einer Ringdichtung 11 mit einem Auflagering 12 auf und ist durch eine den Auflagering 12 und den Ampullenhals 4a umgreifende Bördelkappe 18 fest mit der Zylinderampulle 4 verbunden.

Der Konus 10 besitzt einen axialen Verbindungskanal 13 zum Innenraum der Zylinderampulle 4 hin, so daß dieser mit der Kanüle 1 über das Kupplungsstück 2 zum Durchfluß des Inhaltes der Zylinderampulle 4 verbunden ist.

Der Konus 10 weist im Anschluß an den Auflagering 12 eine mit dem Gewindeteil 8 (dessen Widerlagerfläche 8a) zusammenwirkende Anlagefläche 14 auf, die in bevorzugter Weise von einem Einsteckkonus gebildet ist, der in eine konische Bohrung 8b des Gewindeteil-Topfbodens 8a einfaßt, so daß dadurch die Zylinderampulle 4 in Richtung Kanüle 1 lagefixiert ist.

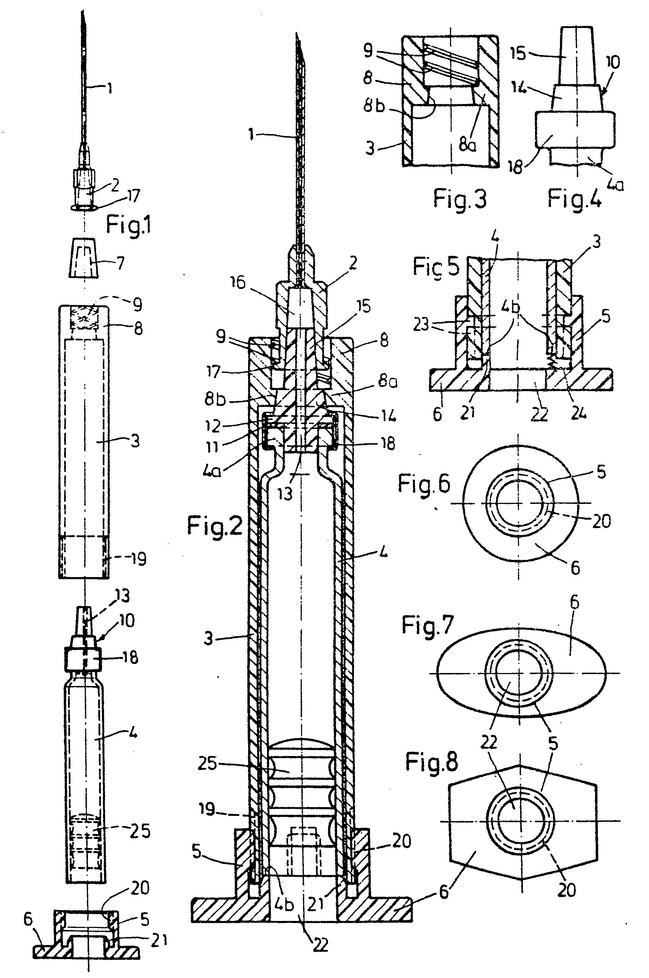

An diesen Einsteckkonus 14 schließt sich ein Aufsteckkonus 15 an, welcher gegenüber dem Einsteckkonus 14 im Querschnitt verringert, das heißt gegenüber dem Eisntecckonus 14 abgestuft ist, und der das freie Längenende des Konusses 10 bildet und über die Stirnfläche des Spritzenkörpers 3 im eingesetzten Zustand der Zylinderampulle 4 um ein gewisses Maß hinausragt (vgl. Fig. 2).

Das mit der Kanüle 1 fest verbundene Kupplungsstück 2 besitzt einen formschlüssig den Aufsteckkonus 15 übergreifenden Innenkonus als Dichtkonus 16, und dieses Kupplungsstück 2 greift mit einem Teilbereich seiner Länge in das Gewindeteil 8 ein und besitzt einen außen vorstehenden Befestigungsflansch 17, der mit dem Gewinde 9 zusammenwirkt.

Das dem Konus 10 gegenüberliegende Längenende der Zylinderampulle 4 ist durch einen mittels eines nicht dargestellten Stempels zum Herausdrükken des Ampulleninhaltes, vorzugsweise Flüssigkeit oder Paste, verschiebbaren Kolben 25 verschlossen.

Die Zylinderampulle 4 liegt einenends mit ihrem Konus 10 im Bereich des Gewindeteiles 8 lagefixiert im Spritzenkörper 3 und ist anderenends gegen axiales Verschieben in dem Spritzenkörper 3 gehalten, und zwar in bevorzugter Weise durch eine Fixierhülse 5 mit Fingerauflage 6, die durch Aufschrauben, durch Bajonettverschluß, durch Rastverbindung oder dergleichen mit dem Spritzenkörper 3 verbindbar ist, so daß durch diese beiden Anlagestellen 8b, 14 und 3, 5 die eingesetzte Zylinderampulle 4 in dem Spritzenkörper 3 lagemäßig festgesetzt ist.

Gemäß der Ausführung nach Fig. 1 und 2 ist das dem Gewindeteil 8 abgewendete Längenende des Spritzenkörpers 3 mit einem Außengewinde 19 ausgestattet, auf die die Fixierhülse 5 mit einem Innengewinde 20 aufgeschraubt wird. Innenseitig weist die Fixierhülse 5 eine auf das freie Stirnende 4b der Zylinderampulle 4 einwirkende Druckfläche 21, vorzugsweise einen angeformten Druckring, auf, durch den die Zylinderampulle 4 mit ihrem Einsteckkonus 14 (Anlagefläche) fest gegen die konische Bohrung 8b (Widerlagerfläche) gedrückt wird.

Die Fixierhülse 5 bildet einen den Druckring 21 durchziehenden Durchgang 22 für den mit dem Kolben 25 durch Schraubverbindung verbindbaren Stempel.

Gemäß der weiteren Ausführung nach Fig. 5 ist die Fixierhülse 5 mit dem Spritzenkörper 3 durch einen Bajonettverschluß 23, dessen zusammenwirkenden Teile (Nuten und Vorsprünge) an der Fixierhülse 5 innenseitig und an dem Spritzenkörper 3 außenseitig vorgesehen sind, lösbar verbunden, wobei hierbei die Fixierhülse 5 ebenfalls mit einem Druckring 21 auf die Ampullen-Stirnfläche 4b direkt einwirken kann (vgl. linke Hälfte der Fig. 5).

Weiterhin besteht die Möglichkeit, die Fixierhülse 5 durch eine Rastverbindung (Nut-Wulst-Verbindung) am Spritzenkörper 3 festzulegen, wobei die zusammenwirkenden Rastmittel ebenfalls wechselweise an der Fixierhülse 5 innenseitig und an dem Spritzenkörper 3 außenseitig vorgesehen sind.

Zwischen Druckfläche 21 der Fixierhülse 5 und der Ampullen-Stirnfläche 4b kann zusätzlich bei den verschiedenen Befestigungsarten der Fixierhülse 5 eine Druckfeder 24 eingesetzt sein (vgl. rechte Hälfte der Fig. 5).

Die Fingerauflage 6 kann die verschiedensten Grundformen, wie Kreisform (Fig. 6), Ovalform (Fig. 7), Mehreckform (Fig. 8) oder dergleichen, haben. Die Fingerauflage 6 ist mit der Fixierhülse 5 und der Druckfläche 21 einteilig aus Kunststoff hergestellt.

Ebenfalls ist der Luer-Lock-Konus 10 der Zylinderampulle 4 einteilig aus Kunststoff hergestellt, und das Kupplungsstück 2 mit der Kanüle 1 besteht aus Metall oder Kunststoff.

In den Spritzenkörper 3 wird herstellungsseitig die gefüllte Zylinderampulle 4 eingesetzt, die mit ihrer Mantelwandung mit gewissem Spiel zur Mantelwandung des Spritzenkörpers 3 liegt und die mit ihrem Konus 10 in das Gewindeteil 8 fixiert eingreift, wobei der Einsteckkonus 14 zentriert in der konischen Bohrung 8b liegt. Dann wird auf das andere Ende des Spritzenkörpers 3 die Fixierhülse 5 aufgeschraubt (oder durch Bajonettverschluß oder durch Rastverbindung befestigt), wobei die Druckfläche 21 gegen das Stirnende 4b drückt und somit die Zylinderampulle 4 zwischen Konushalterung 14, 8b und Druckfläche 21 fest gehalten wird.

Der Aufsteckkonus 15 der Zylinderampulle 4 ragt in Axialrichtung aus dem Gewindeteil 8 des Spritzenkörpers 3 heraus, und sein Verbindungskanal 13 ist durch den abnehmbaren Verschlußstopfen 7 verschlossen. Der so komplettierte Spritzenkörper 3 wird dann mit der Kanüle 1 steril verpackt.

Beim Gebrauch der Spritze nimmt der Benutzer den Verschlußstopfen 7 ab und steckt die Kanüle 1 mit ihrem Kupplungsstück 2 auf den Aufsteckkonus 15, und dann dreht der Benutzer das Kupplungsstück 2 axial, so daß der Befestigungsflansch 17 des Kupplungsstückes 2 mit dem Gewinde 9 zusammenwirkt und das Kupplungsstück 2 den Aufsteckkonus 15 fest in seinen Dichtkonus 16 hineinzieht, so daß eine dichte Verbindung zwischen beiden Teilen 2, 15 erfolgt. Die Zylinderampulle 4 kann sich bei dieser Luer-Lock-Verbindung im Spritzenkörper 3 durch ihre beidendseitige und eingespannte Fixierung im Spritzenkörper 3 nicht bewegen, und dadurch wird eine dichte und hohe Verbindungsfestigkeit der Kanüle 1 am Konus 10 (15) erreicht.

Gleichzeitig beim Aufsetzen der Kanüle 1 ist eine Verbindung des Zylinderampullen-Innenraumes mit der Kanüle 1 durch den im Konus 10 vorgesehenen Verbindungskanal 13 geschaffen, ohne daß ein Dichtelement oder dergleichen durchstochen werden muß.

Es liegt im Rahmen der Erfindung, auch die plattenförmige Fingerauflage 6 direkt als Druckfläche 21 zu benutzen, so daß kein vorstehender Druckring 21 anzuformen ist; hierbei müßte jedoch die Zylinderampulle mit ihrem Stirnende 4b um ein gewisses Maß aus dem Spritzenkörper 3 herausragen.

**Patentansprüche**

1. Injektionsspritze, bestehend aus einem Spritzenkörper (3) und einer darin eingesetzten Zylinderampulle (4), mit einem zweiteilig ausgebildeten Luer-Lock-Anschluß (8, 10) für eine Kanüle (1), welcher Luer-Lock-Anschluß aus einem an der Zylinderampulle (4) angeordneten Konus (10) und aus einem an einem Ende des Spritzenkörpers vorgesehenen Gewindeteil (8) gebildet ist, wobei der Konus (10) das Gewindeteil (8) axial durchgreift, dadurch gekennzeichnet, daß ein den Konus (10) axial durchsetzender, sich bis zum Innenraum der Zylinderampulle erstreckender Verbindungskanal (13) vorgesehen ist, daß der Verbindungskanal (13) an seinem der Zylinderampulle abgewandten Ende mit einem abnehmbaren Verschlußstopfen (7) verschlossen ist und daß die Zylinderampulle (4) an dem einen Ende mit einer Anlagefläche (14) des Konus (10) im Gewindeteil (8) lagefixiert und mit dem anderen Ende im Spritzenkörper (3) gegen axiale Verschiebung gesichert ist.

2. Injektionsspritze nach Anspruch 1, dadurch gekennzeichnet, daß der Spritzenkörper (3) von einem Rohr mit kreisförmigem Querschnitt aus Kunst-stoff, vorzugsweise durchsichtigem Kunststoff, ge-bildet ist und ein Rohr-Längenende das Gewindeteil (8) des Luer-Lock-Anschlusses ergibt, welches hülsenförmig mit einem Innengewinde (9) ausgebildet ist und welches innenseitig einen Boden (8a) als Anlagefläche für die Zylinderampulle (4) aufweist.

3. Injektionsspritze nach Anspruch 1 und 2, dadurch gekennzeichnet, daß der Konus (10) des ampullenseitigen Luer-Lock-Anschlusses in sich abgestuft ist und einen sich an den Ampullenhals (4a) anschließenden Einsteckkonus (14) als Anlagefläche und einen sich daran fortsetzenden, verkleinerten und aus dem Gewindeteil (8) des Spritzenkörpers (3) axial herausragenden Aufsteckkonus (15) zur Aufnahme des lösbaren Verschlußstopfens (7) und zum Aufsetzen eines Kupplungsstückes (2) der Kanüle (1) aufweist.

4. Injektionsspritze nach den Ansprüchen 1 bis 3, dadurch gekennzeichnet, daß im Boden (8a) des Gewindeteiles (8) zentrisch eine konische Bohrung (8b) ausgespart ist, in die der ampullenseitige Einsteckkonus (14) lagefixiert einfaßt und diese beiden ineinanderfassenden Konusflächen die Ampullen-Anlagefläche (14) und Ampullen-Widerlagerfläche (8b) bilden.

5. Injektionsspritze nach den Ansprüchen 1 bis 4, dadurch gekennzeichnet, daß das Kupplungsstück (2) der Kanüle (1) in bekannter Weise einen den Aufsteckkonus (15) der Zylinderampulle (4) überfassenden Dichtkonus (16) hat und mit einem Befestigungsflansch (17) mit dem Gewinde (9) des Gewindeteiles (8) am Spritzenkörper (3) zusammenwirkt.

6. Injektionsspritze nach den Ansprüchen 1 bis 5, dadurch gekennzeichnet, daß auf das dem Gewindeteil (8) gegenüberliegende Längenende des Spritzenkörpers (3) eine mit einer Druckfläche (21) auf die Stirnseite (4b) der Zylinderampulle (4) einwirkende und die Zylinderampulle (4) mit ihrem Einsteckkonus (14) in die konische Bohrung (8b) drükkende Fixierhülse (5) mit angeformter Fingerauflage (6) lösbar festgelegt ist.

7. Injektionsspritze nach Anspruch 6, dadurch gekennzeichnet, daß die Fixierhülse (5) durch Schrauben, Bajonettverschluß, Rastverbindung oder dergleichen mit dem Spritzenkörper (3) verbunden ist.

8. Injektionsspritze nach den Ansprüchen 6 und 7, dadurch gekennzeichnet, daß die Fixierhülse (5) mit einem Innengewinde (20) auf ein Außengewinde (19) des Spritzenkörpers (3) aufschraubbar ist und innenseitig einen vorstehenden, umlaufenden und gegen die Ampullen-Stirnfläche (4b) einwirkenden, angeformten Druckring (21) aufweist.

9. Injektionsspritze nach den Ansprüchen 6 und 7, dadurch gekennzeichnet, daß die Fixierhülse (5) innenseitig und der Spritzenkörper (3) außenseitig mit zusammenwirkenden Gegenstücken wie Nuten und Vorsprüngen eines Bajonettverschlusses (23) ausgestattet sind und die Fixierhülse (5) eine gegen die Ampullen-Stirnfläche (4b) einwirkende, ringförmige Druckfläche (21) aufweist.

10. Injektionsspritze nach Anspruch 9, dadurch gekennzeichnet, daß zwischen Fixierhülse (5) und Stirnfläche (4b) der Zylinderampulle (4) eine Druckfeder (24) angeordnet ist.

11. Injektionsspritze nach den Ansprüchen 1 bis 10, dadurch gekennzeichnet, daß die Fixierhülse (5) mit Fingerauflage (6) und Druckfläche (21) einteilig aus Kunststoff hergestellt ist und die Fingerauflage (6) eine kreisförmige, ovale oder eckige Grundform mit einem zentralen Durchlaß (22) für einen mit dem Kolben (25) der Zylinderampulle (4) zusammenwirkenden Stempel besitzt.

12. Injektionsspritze nach den Ansprüchen 1 bis 5, dadurch gekennzeichnet, daß der Konus (10) des Luer-Lock-Anschlusses einteilig aus Kunststoff besteht, in den Ampullenhals (4a) eingesteckt ist und mit einem Auflagering (12) unter Zwischenschaltung eines Dichtringes (11) auf dem Ampullenhals (4a) aufliegt sowie durch eine Bördelkappe (18) fest mit der Zylinderampulle (4) verbunden ist.

## Claims

1. An injection syringe, comprising a syringe body (3) and a cylindrical ampoule (4) inserted therein, with a twopart Luer-lock connection (8, 10) for a cannula (1), which Luer-lock connection is formed by a cone (10) arranged on the cylindrical ampoule (4) and by a threaded part (8) provided at one end of the syringe body, the cone (10) axially penetrating the threaded part (8), characterised in that a connecting passage (13) is provided which passes axially through the cone (10) and extends as far as the interior of the cylindrical ampoule, in that the conncting passage (13) is closed by a detachable plug (7), and in that at one end the cylindrical ampoule (4) is held in position in the threaded part (8) by a bearing surface (14) of the cone (10) and at its other end is secured against axial displacement in the syringe body (3).

2. An injection syringe according to Claim 1, characterised in that the syringe body (3) comprises a tube of circular cross-section and consisting of plastics material, preferably transparent plastics material, and at one longitudinal end of the tube provides the threaded part (8) of the Luer-lock connection, which is sleeve-shaped with an internal thread (9) and which on the inside has a base (8a) as a bearing surface for the cylindrical ampoule (4).

3. An injection syringe according to Claims 1 and 2, characterised in that the cone (10) of the Luer-lock connection on the ampoule side is stepped per se and adjoining the ampoule neck (4a) has an insert cone (14) as a bearing surface and, in extension thereof, a reduced mounting cone (15) projecting axially from the threaded part (8) of the syringe body (3) for accomodating the releasable plug (7) and for fitting a coupling member (2) of the cannula (1).

4. An injection syringe according to Claims 1 to 3, characterised in that a tapered bore (8b) is provided centrally in the base (8a) of the threaded part (8), the insert cone (14) on the ampoule side engaging in said bore so as to be held in position and these two interengaging conical surfaces form the ampoule bearing surface (14) and ampoule abutment surface (8b).

5. An injection syringe according to Claims 1 to 4, characterised in that the coupling member (2) of the cannula (1) has in known manner a sealing cone (16) engaging over the mounting cone (15) of the cylindrical ampoule (4) and with a mounting flange (17) cooperates with the thread (9) of the threaded part (8) on the syringe body (3).

6. An injection syringe according to Claims 1 to 5, characterised in that a securing sleeve (5) with an integral finger support (6) is detachably fastened to the longitudinal end of the syringe body (3) opposite the threaded part (8), which securing sleeve acts on the end face (4b) of the cylindrical ampoule (4) with a thrust surface (21) and urges the cylindrical ampoule (4) with its insert cone (14) into the tapered bore (8b).

7. An injection syringe according to Claim 6, characterised in that the securing sleeve (5) is connected to the syringe body (3) by screw connection, bayonet fixing, detent connection or the like.

8. An injection syringe according to Claims 6 and 7, characterised in that the securing sleeve (5) can be screwed with an internal thread (20) on to an external thread (19) of the syringe body (3) and on its inside has an integral protruding annular thrust ring (21) acting against the end surface (4b) of the ampoule.

9. An injection syringe according to Claims 6 and 7, characterised in that the inside of securing sleeve (5) and the outside of the syringe body (3) are provided with cooperating counter members, such as grooves and projections of a bayonet fixing (23), and the securing sleeve (5) has an annular thrust surface (21) acting against the end surface (4b) of the ampoule.

10. An injection syringe according to Claim 9, characterised in tha a compression spring (24) is disposed between the securing sleeve (5) and the end surface (4b) of the cylindrical ampoule (4).

11. An injection syringe according to Claims 1 to 10, characterised in that securing sleeve (5), with finger support (6) and thrust surface (21), is made in one piece from plastics material and the finger support (6) has of circular, oval or angular basic shape with a central opening (22) for a plunger cooperating with the piston (25) of the cylindrical ampoule (4).

12. An injection syringe according to Claims 1 to 5, characterised in that the cone (10) of the Luer-lock connection is made in one piece from plastics material, is inserted into the ampoule neck (4a) and bears against the ampoule neck (4a) with a bearing ring (12) with interposition of a sealing ring (11), and is securely connected to the cylindrical ampoule (4) by a flanged cap (18).

## Revendications

1. Seringue d'injection composée du corps de la seringue (3) et d'une ampoule cylindrique (4) logée à l'intérieur de celui-ci, avec un raccordement Luer-Lock en deux pièces (8, 10) pour une canule (1), lequel raccordement Luer-Lock est formé par un cône (10) centré sur l'ampoule cylindrique (4) et par une pièce filetée (8) prévue à l'extrémité du corps de la seringue, le cône (10) étant saisi axialement par la pièce filetée, seringue d'injection caractérisée par le fait qu'un canal de liaison (13), traversant axialement le cône (10) est prévu, s'étendant jusqu'à l'intérieur de l'ampoule cylindrique, et que le canal de liaison (13) est fermé, à son extrémité opposée à l'ampoule cylindrique, à l'aide d'un bouchon de fermeture amovible (7), et que l'ampoule cylindrique (4) est fixée en position voulue, à une extrémité, avec une surface de contact (14) du cône (10), dans la pièce filetée (8) et assurée, à l'autre extrémité, contre tout déplacement axial dans le corps de la seringue (3).

2. Seringue d'injection selon spécification 1, caractérisée par le fait que le corps de la seringue (3) est constitué par un tube de section circulaire en matière plastique, de préférence en matière plastique transparente, et qu'une extrémité longitudinale du tube forme la pièce filetée (8) du raccordement Luer-Lock, pièce filetée en forme de douille à filetage intérieur (9) et présentant vers l'intérieur un fond (8a) servant de surface de contact à l'ampoule cylindrique (4).

3. Seringue d'injection selon spécifications 1 et 2, caractérisée par le fait que le cône (10) du raccordement Luer-Lock présente des gradins en lui-même côté ampoule et comprend un cône enfichable (14) faisant suite au goulot de l'ampoule (4a) et servant de surface de contact, ainsi qu'un cône d'emboîtement (15) en prolongement, lequel s'amincit et, faisant axialement saillie hors de la pièce filetée (8) du corps de la seringue (3), est destiné à recevoir le bouchon de fermeture amovible (7), et sur lequel s'emboîte la pièce de couplage (2) de la canule (1).

4. Seringue d'injection selon les spécifications 1 à 3, caractérisée par le fait qu'une forure conique (8b) centrée est pratiquée dans le fond (8a) de la pièce filetée (8), forure dans laquelle le cône (14), enfichable côté ampoule, est encastré en position fixe, et que ces deux surfaces coniques s'emboîtant l'une dans l'autre forment la surface de contact (14) et la surface de butée (8b) de l'ampoule.

5. Seringue d'injection selon les spécifications 1 à 4, carctérisée par le fait que la pièce de couplage (2) de la canule (1) est pourvue, de manière connue, d'un cône d'étanchéité (16) surmontant le cône d'emboîtement (15) de l'ampoule cylindrique (4), et agit, avec un flasque de fixation (17), en concours avec le filetage (9) de la pièce filetée (8) du corps de la seringue (3).

6. Seringue d'injection selon les spécifications 1 à 5, caractérisée par le fait qu'une douille de fixation (5) avec contact doigt formé (6) est fixée de façon amovible sur l'extrémité longitudinale du corps de la seringue (3), à l'opposé de la pièce fileté (8), laquelle douille de fixation agit avec une surface de pression (21) sur la partie frontale (4b) de l'ampoule cylindrique (4) et presse son cône enfichable dans la forure conique (8b).

7. Seringue d'injection selon spécification 6, caractérisée par le fait que la douille de fixation (5) est reliée au corps de la seringue (3) par vis, fermeture à baïonnette, fermeture à cran ou autre dispositif analogue.

8. Seringue d'injection selon spécification 6, caractérisée par le fait que la douille de fixation (5) est vissable à l'aide de son filetage intérieur (20) sur un filetage extérieur (19) du corps de la seringue (3) et présente, à l'intérieur, une bague de pression formée (21) tournante, faisant saillie et agissant contre la surface frontale (4b) de l'ampoule.

9. Seringue d'injection selon les spécifications 6 et 7, caractérisée par le fait que l'intérieur de la douille de fixation (5) et l'extérieur du corps de la seringue (3) sont équipés de contre-pièces agissant de concours, telles que rainures et saillies d'une fermeture à baïonette (23), et que la douille de fixation (5) présente une surface de pression (21) annulaire, agissant sur la surface frontale (4b) de l'ampoule.

10. Seringue d'injection selon spécification 9, caractérisée par le fait qu'un ressort (24) est disposé entre la douille de fixation (5) et la partie frontale (4b) de l'ampoule cylindrique (4).

11. Seringue d'injection selon spécifications 1 à 10, caractérisée par le fait que la douille de fixation (5) avec contact doigt (6) et surface de pression (21) sont exécutés d'une pièce en matière plastique, et que la surface contact doigt (6) est pourvue d'une pièce de base ronde, ovale ou angulaire avec passage central (22) pour la tige du piston (25) de l'ampoule cylindrique (4).

12. Seringue d'injection selon les spécifications 1 à 5, caractérisée par le fait que le cône (10) du raccordement Luer-Lock est exécuté d'une pièce, en matière plastique, est enfiché dans le goulot de l'ampoule (4a) et, pourvu d'un bague de contact (12), une bague d'étanchéité (11) étant intercalée, repose sur le col de l'ampoule (4a), et est relié fixement à l'ampoule cylindrique (4) par une couronne (18).

Fig.1

Fig.2

Fig.3

Fig.4

Fig.5

Fig.6

Fig.7

Fig.8